# EUROPEAN PATENT SPECIFICATION

(11) **EP 4 232 143 B1**
(45) Date of publication and mention of the grant of the patent: **23.10.2024**
(21) Application number: 21805372.6
(22) Date of filing: 19.10.2021
(51) Int. Cl.: A61N 2/00, A61N 2/02

(54) **AN ELECTROMAGNETIC TREATMENT DEVICE**
ELEKTROMAGNETISCHE BEHANDLUNGSVORRICHTUNG
DISPOSITIF DE TRAITEMENT ÉLECTROMAGNÉTIQUE

(30) Priority: 22.10.2020 DK PA202070696
(43) Date of publication of application: 30.08.2023
(73) Proprietor: MoodHeadBand ApS, 3000 Helsingør (DK)
(72) Inventor: VINDELØV, Anne Vibeke, 3000 Helsingør (DK); HANSEN, Jørgen Borup, 3000 Helsingør (DK)
(74) Representative: AWA Denmark A/S
(86) International application number: PCT/DK2021/050310
(87) International publication number: WO 2022/083837

(56) References cited:
- EP-A1- 3 517 159
- WO-A2-2008/051790
- WO-A2-2010/100643
- WO-A2-2015/155605
- DE-U1- 20 305 191

## Description

### Technical Field

The present invention relates to an electromagnetic device adapted for stimulating tissue, particularly brain tissue, of a subject with pulsed electromagnetic fields (PEMF) with the aim of treating psychiatric disorders, in particular depression disorders and/or sleep disorders, the electromagnetic device comprising a band-shaped body structure, a plurality of coils and drive electronics.

### Background Art

Depression is an increasingly wide spread suffering or disorder, which far too often remains sparsely treated or un-treated altogether.

Many years ago, treatment of severe depression with electroshock was developed. This treatment has proved highly efficient for even severe depressions and at times the treatment may be life-saving. This treatment is carried out by sending a forceful pulsing direct current bilaterally through the subject's cranium. However, before commencing such treatment it is necessary to sedate the subject and ensure that the subject's muscles are fully relaxed such as to avoid the risk of cramping, which may be severe enough to cause fractures. Therefore, such treatment must take place in a hospital under medical surveillance. Furthermore, electroshock treatment may have severe side effects, for instance in the form of effects on the subject's memory, which may be severe enough to erase periods of the subject's life from memory. Due to the side effects and possible complications, treatment by electroshock is not widely used.

More recently experiments and treatments using electromagnetic fields to affect the brain have been conducted and developed. One example is a method known as TMS or Transcranial Magnetic Stimulation according to which the brain is treated with forceful magnetic fields. TMS does not require sedation, nor does it affect the memory adversely. However, the magnetic fields have an intensity of 1-2 Tesla and therefore requires very costly equipment. Furthermore, as there is a risk of epileptic cramps or seizures occurring as a side effect, this treatment must take place in a hospital under medical surveillance. Furthermore, many treatment sessions are required to obtain the desired effect, and therefore the treatment is also costly. Finally, the treatment is unpleasant while taking place due to noise, and due to that the forceful magnetic fields may invoke reflectory convulsions in the muscles, which may cause soreness and headache.

Treatment with weaker magnetic fields as compared to those used in TMS is also known, albeit primarily for wellness purposes. The use of weaker magnetic fields has the advantage of avoiding the above described side effects.

One such treatment of depression disorders using weaker magnetic fields as compared to those used in TMS has recently become known. This treatment is a method known as T-PEMF or Transcranial Pulsing ElectroMagnetic Fields. This method is something between TMS methods and PEMF or Pulsing ElectroMagnetic Fields methods. Methods using T-PEMF is known from e.g. US 2012/101327 A1 and US 6,561,968 B1. According to the manufacturer this treatment may only be conducted in psychiatric clinics and the equipment is not sold but must be hired at the manufacturers.

WO 2015/155605 A1 discloses a headband, in which one or more inductive coils is built in. The inductive coils are connected to a GSM device by a cable. The GSM device 1 functions as a source of power and a source of emitting the pulsating frequencies.

WO 2008/051790 A2 discloses a magnetic field generator with a coil and a power source and a microcontroller in electrical communication with the power source and the coil. The coil generates a pulsed magnetic wave of an alternating current when a sensor determines that the magnetic field generator is located in proximity to a living being. The magnetic field generator is structured to be coupled to a device and the device is structured to be worn by the living being in proximity to the living being.

EP 3 517 159 A1 discloses a brain stimulating device comprising a brainwave measurement unit for outputting a brainwave signal and a stimulation unit for applying spindle-like stimulation to a brain in accordance with the generation of slow oscillation included in the brainwave signal.

DE 203 05 191 discloses an apparatus comprising a head part with a current distributor comprising a number of wires carrying current to wave generators. The wave generators include respective housing and cover, forming an intermediate space through which a headband can be fed.

WO 2010/100643 A2 discloses methods for improved neuron excitation, the methods comprising subjecting one or more neurons to a rotating electric field, thereby exciting said neuron or said neurons. The rotating electric field is the resultant sum of at least two time-dependent electric fields. The at least two time-dependent electric fields are induced by at least two time-dependent magnetic fields. The at least two time-dependent magnetic fields are induced by passing currents through at least two separate and independent coils.

Furthermore, WO 2016/127183 A1 discloses and apparatus for treating subjects with directionally focussed energy. A broad range of possible energy pulses and disorders that may be treated is disclosed. The apparatus may be shaped as a head-band or as a pillow. The apparatus includes a pair of coils arranged on either side of the forehead of the subject. When shaped as a head-band, the apparatus is kept in place on the head of the subject by means of two strips extending over the crown of the subject's head. The apparatus further comprises a controller, an energy source, biometric measurement devices and energy emitting portals arranged in an array. The energy emitting portals ensure that the energy to which the subject is subjected is directed in a desired direction.

There is a desire for providing an electromagnetic treatment device for home use with which treatment using PEMF or Pulsing Electromagnetic Fields methods become possible.

There is also a desire for providing such an electromagnetic treatment device which may become more wide spread, and which is thus cost effective in production and thus cheap, and which is more simpler and easier to use in particular for the subject as compared to existing devices such as those described above.

### Summary of Invention

It is therefore the object of the invention to provide an electromagnetic treatment device for home use with which treatment using PEMF or Pulsing Electromagnetic Fields methods become possible.

It is a further object of the invention to provide such an electromagnetic treatment device which is also cost effective in production and thus cheap, and which is simpler and easier to use in particular for the subject as compared to existing devices such as those described above.

These and other objects are achieved by means of an electromagnetic device adapted for stimulating tissue, particularly brain tissue, of a subject with pulsed electromagnetic fields (PEMF) with the aim of treating psychiatric disorders, particularly depression disorders and/or sleep disorders, the electromagnetic device comprising a band-shaped body structure, a plurality of coils and drive electronics, the drive electronics being configured for driving the plurality of coils by providing alternating current, AC, pulses to the plurality of coils, the plurality of coils being configured for, in operation and in reaction to the AC pulses received from the drive electronics, providing a stimulus in the form of PEMFs to the tissue of a subject, where the plurality of coils is provided in and/or on the band-shaped body structure distributed along a length of the band-shaped body structure such that when the band-shaped body structure is mounted on the head of a subject in a head-band-like manner, coils are arranged along all of the circumference of the head of the subject, and such that at least one coil of the plurality of coils is arranged adjacent to the frontal region of the brain of the subject such as to enable, when the coils are in operation, stimulating the frontal region of the brain of the subject and that other coils of the plurality of coils are arranged adjacent to other regions of the brain such as to enable, when the coils are in operation, stimulating other regions of the brain of the subject than the frontal region, and where the drive electronics is configured to operate the plurality of coils such that coils, which when the band-shaped body structure is mounted on the head of a subject are arranged on mutually opposite positions of the head of the subject, are operated in mutually opposite phases.

The frontal regions of the brain have been recognized as playing an important role in especially depression and sleep disorders. Based on this recognition, the inventor has proven that by providing the plurality of coils such that when the band-shaped body structure is mounted on the head of a subject in a head-band-like manner, and particularly in a head-band-like manner where the band-shaped body structure extends along all of the circumference of the head of the subject, coils are arranged along all of the circumference of the head of the subject, and such that at least one coil of the plurality of coils is arranged adjacent to the frontal region of the brain of the subject such as to enable, when the coils are in operation, stimulating the frontal region of the brain of the subject provides for an electromagnetic treatment device with an improved effect when treating the subject's brain, especially for depression disorders and sleep disorders. It is noted that as used herein, the frontal regions of the brain comprise the frontal lobe, and in particular the frontal parts of the frontal lobe, such as for instance the prefrontal cortex.

Thereby, an improved electromagnetic treatment device for home use with which treatment using PEMF or Pulsing Electromagnetic Fields-methods has been provided. Also, such an electromagnetic treatment device is simpler and easier to use in particular for the subject as compared to existing devices such as those described above.

Also, the mutually opposite coils provide an amplification of each other's magnetic field and thus of the PEMFs used to stimulate the subject's brain. Thereby a more efficient electromagnetic device is provided for.

In an embodiment, the coils of the plurality of coils are distributed evenly along the length of the band-shaped body structure.

Thereby, an electromagnetic treatment device is provided with which treatment may be administered evenly around the head of a subject when used as a head-band, including the forehead region of the subject.

In an embodiment, the coils of the plurality of coils are distributed along the entire length of the band-shaped body structure.

Thereby, an electromagnetic treatment device is provided with which treatment may be administered around the head of a subject when used as a head-band with a focus on desired areas or regions of the brain, including the frontal region of the brain of the subject.

In an embodiment, the band-shaped body structure comprises opposite end sections at longitudinally opposite ends, where no coils are arranged in the end sections.

Thereby, free end sections are provided for. Such free end sections may be used for instance to make room for an arrangement making it possible to attach the end sections releasably to one another and thus to mount the band-shaped body structure on the head of a subject to be treated, or to incorporate the band-shaped body structure in a pillow in such a way that correct alignment with a head of a subject is obtained when the subject lies his or her head on the pillow.

In an embodiment, the drive electronics are configured to operate at an impedance of 1 to 16 Ohms or 4 to 16 Ohms.

Thereby, an electromagnetic treatment device is provided where the drive electronics operate at impedances corresponding to commonly used loudspeakers, which may thus be used to provide the audio input used to generate the AC pulses. Thereby, an electromagnetic treatment device is provided where the drive electronics may consist of commonly available electronic components which are cheap. Such an electromagnetic treatment device is thus also cost effective in production and thus cheap to produce.

In an embodiment, the drive electronics are configured to operate at a voltage below 25 Volts.

Thereby, an electromagnetic treatment device is provided where the drive electronics operate at such a voltage that commonly applicable rules relating to user safety of domestic electronic equipment are fulfilled. An example of such rules is Directive 2014/35/EU of the European Parliament and of the Council of 26 February 2014 on the harmonization of the laws of the Member States relating to the making available on the market of electrical equipment designed for use within certain voltage limits. Thereby, the security of the subject is also secured.

In an embodiment, the drive electronics is configured to operate the plurality of coils such that coils which are spaced apart with a distance corresponding to half of a length of the band-shaped body structure are operated in mutually opposite phases.

Thereby, the mutually opposite coils may provide an amplification of each other's magnetic field and thus of the PEMFs used to stimulate the subject's brain. Thereby a more efficient electromagnetic device is provided for.

In an embodiment, the drive electronics is configured to provide the plurality of coils with AC-pulses in the form of square pulses. Alternatively, the drive electronics is configured to provide the plurality of coils with AC-pulses in the form of saw-tooth pulses.

Providing square pulses with a period of less than one second, such as in the millisecond range, e.g. less than 6 ms, and a rise time of less than one second has the further advantage of allowing the electromagnetic device to cool down sufficiently between PEMFs to predominantly or completely avoid overheating, which may otherwise cause damage to not only the electromagnetic device but also the subject.

In an embodiment, the drive electronics is configured to provide the plurality of coils with AC pulses having a frequency of less than 3 Hz, particularly 1,5 to 3 Hz.

AC pulses with such frequencies are very similar to the frequencies of the waves generated by the human brain while asleep and are therefore especially suitable for generating PEMFs which are particularly efficient for simulating brain waves generated by the brain when asleep. Thereby, an electromagnetic device especially efficient for treating sleep disorders is provided for.

In an embodiment, the drive electronics is configured to provide the plurality of coils with AC pulses having a frequency of between 45 and 60 Hz, particularly 50 to 60 Hz, such as about 55 Hz.

AC pulses with such frequencies have been shown to generate PEMFs which are particularly efficient for treating depression disorders. Thereby, an electromagnetic device especially efficient for treating depression disorders is provided for.

In an embodiment, the band-shaped body structure is made of a resilient and heat resistant material, such as silicone. The plurality of coils may further be embedded in the resilient material.

Making the band-shaped body structure of a resilient and heat resistant material provides for an electromagnetic device which is particularly good for use as a head-band or incorporation in a pillow, and which is furthermore durable. Silicone has the advantage of furthermore being relatively cheap and at the same time non-toxic and skin friendly. Other suitable, and in particular skin friendly, materials may also be envisaged.

In an embodiment, the drive electronics comprises a class d amplifier configured to amplify AC pulses.

Class d amplifiers are advantageous due to having very low losses and an efficiency of 90 % or more. This enables providing an electromagnetic treatment device where the drive electronics has small dimensions and may consist of commonly available electronic components which are relatively cheap. Such an electromagnetic treatment device is thus also cost effective in production and thus cheap to produce. Class d amplifiers are also available with many other useful features already built in. Examples of such built-in features are a communication interface or sender/receiver for e.g. Bluetooth communication and a connection for an USB device or other memory unit.

In an embodiment, the drive electronics comprises a pulse generator configured to generate AC pulses. The pulse generator may be incorporated in the amplifier.

In an embodiment, the drive electronics comprises a sender/receiver unit configured to send and/or receive data signals and/or AC pulses. The sender/receiver unit may be incorporated in the amplifier.

In an embodiment, the drive electronics comprises a memory unit. The memory unit may be a separate unit, or it may be incorporated in the amplifier.

In an embodiment, the drive electronics comprises a data processing device. The data processing unit may be a separate unit, or it may be incorporated in the amplifier.

In an embodiment, the drive electronics comprises input and/or output terminals for any one or more of a memory unit, a pulse generator configured to generate AC pulses and a sender/receiver unit configured to send and/or receive data signals and/or AC pulses. Advantageously, such terminals are incorporated in the amplifier.

In an embodiment, at least one coil of the plurality of coils is provided with an iron core or a ferrite core.

In an embodiment, at least one coil of the plurality of coils is surrounded at least partially by at least one iron element and/or at least one ferrite element.

Both these embodiments provide for a device where the PEMFs used to stimulate the subject's tissue may be amplified considerably in a functionally and structurally very simple manner. Thereby, an improved effect of the treatment may be achieved.

In an embodiment, the plurality of coils comprises an even number of coils, such as 8, 10, 12, 16 or 20 coils.

Although an uneven number of coils may also be envisaged, an even number of coils has the advantage of making it much easier to control the coils such as to provide mutual amplification of the PEMFs of the coils.

In an embodiment, the coils of the plurality of coils are subdivided in a first half and a second half, the first half extending from a middle of the band-shaped body structure towards a first end section of the band-shaped body structure, and the second half extending from the middle of the band-shaped body structure towards a second end section of the band-shaped body structure opposite to the first end section.

In that way the coils may work in a so-called Helmholtz-configuration. Thereby, the first and second half may amplify each other's PEMFs since the total magnetic field is propagating in the same direction with a concentrated intensity and at the same time alternates in direction as controlled by the drive electronics.

In an embodiment, the drive electronics are arranged externally with respect to the band-shaped body structure and the plurality of coils.

Thereby, a device is provided for which has a band-shaped body structure with a lowered weight and with which the currents and electromagnetic fields prevailing in the vicinity of the drive electronics do not interfere with the PEMFs used to stimulate the subject's tissue.

In an alternative embodiment, the drive electronics are integrated in the band-shaped body structure.

Thereby, a more compact device may be provided for.

In an embodiment, the drive electronics are configured for being controlled via a wireless connection. Such a wireless connection may be Bluetooth or the like. The control of the drive electronics may furthermore be performed through a suitable app on a mobile telephone of a tablet or laptop computer or the like.

Thereby a device which is particularly simple to control is provided for. Also, such a device is particularly simple to keep updated as regards its software, as software updates may be downloaded and installed through the wireless connection or in the app used to control the drive electronics.

In an embodiment, the band-shaped body structure is configured for mounting on the head of a subject. The band-shaped body structure may for instance be made as a head-band.

Such a device is particularly simple to mount correctly on the head of a subject and may be mounted by the subject him- or herself.

In an embodiment, the band-shaped body structure is incorporated in a pillow.

Such a device is particularly simple to use for any subject, especially where the aim is to alleviate or treat sleep disorders.

### Brief Description of Drawings

In the following description embodiments of the invention will be described with reference to the schematic drawings, in which
Fig. 1 shows a top view of a band-shaped body structure of an electromagnetic device according to the invention.
Fig. 2 shows perspective view seen from above of a band-shaped body structure of an electromagnetic device according to Fig. 1 and bent towards the shape of a head-band.
Fig. 3 shows a top view mold for a band-shaped body structure of an electromagnetic device according to the invention in which the coil assembly of the electromagnetic device has already been provided.
Fig. 4 shows a close-up on a section of a mold for a band-shaped body structure of an electromagnetic device according to the invention in which the coil assembly of the electromagnetic device has already been provided, ferrite cores and elements also being provided.
Fig. 5 shows a top view of a drive electronics of an electromagnetic device according to the invention.
Fig. 6 shows a perspective view of the electromagnetic device according to Fig. 1 mounted as a head-band on the head of a subject.

### Description of Embodiments

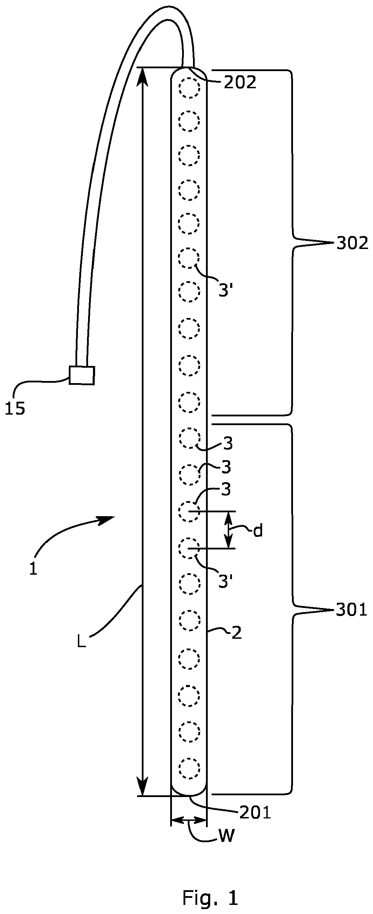

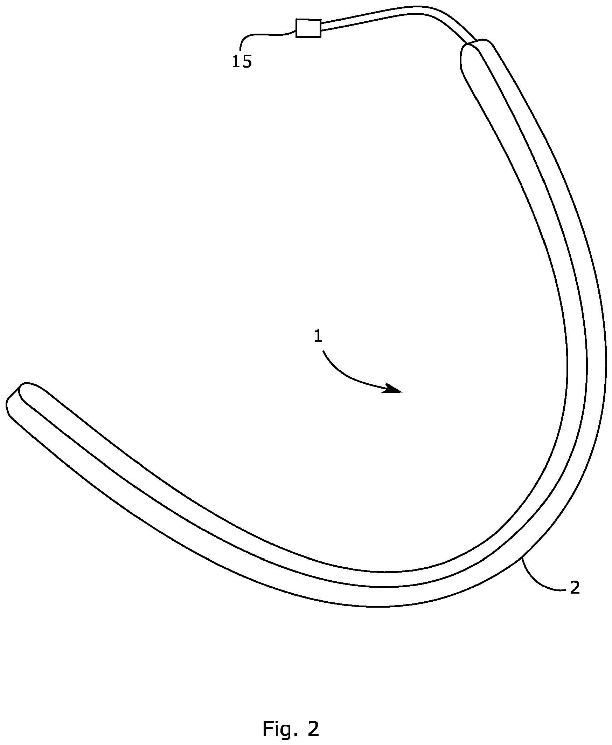

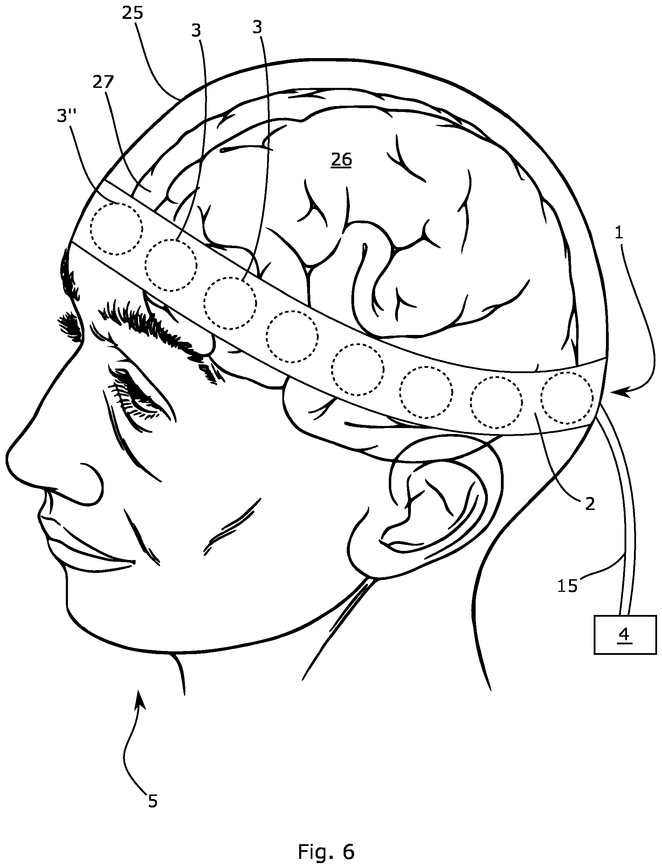

The electromagnetic device 1 shown in Figs. 1, 2 is adapted for stimulating the tissue of a subject 5, and in particular the tissue of the brain 26 of a subject 5, with pulsed electromagnetic fields (PEMF). The aim is to treat psychiatric disorders, such as depression disorders and/or sleep disorders. Fig. 6 shows the electromagnetic device 1 when mounted as a head-band on the head 25 of a subject 5. The electromagnetic device 1 according to the invention may also be used with the aim of treating other disorders.

It is noted that since treatment of the brain with PEMF has also proven advantageous in connection with other disorders, the electromagnetic device 1 according to the invention may also be used for treating other brain related disorders. Examples of such other disorders may be manic-depressive disorders, brain-related motoric disorders such as Parkinson's disease and memory-related disorders such as dementia and Alzheimer's disease.

The electromagnetic device 1 generally comprises a band-shaped body structure 2, a plurality of coils 3 and drive electronics 4.

The band-shaped body structure 2 is generally made of a flexible, heat-resistant and electrically isolating material. Preferably, the material of the band-shaped body structure 2 is also skin friendly and non-toxic. For instance, the band-shaped body structure 2 may be made of a suitable type of silicone or rubber silicone. Such a material has the further advantage of also being easy to disinfect. The band-shaped body structure 2 may thus be bendable - cf. Fig. 2 - such that it can be mounted on or around the head of a subject in the same way as a head-band.

The band-shaped body structure 2 may be arranged in a pouch or cover provided with zipper or other suitable closure. Also, the band-shaped body structure 2 or the pouch or cover may be provided with a closure mechanism, such as hook and loop fasteners or pushbuttons, enabling the opposing longitudinal ends 201, 202 of the band-shaped body structure 2, or the corresponding opposing longitudinal ends of the pouch or cover, to be attached to one another such that it may be used as a head-band (cf. Fig. 6). The pouch or cover may be made of a material being comfortable to wear for the subject and/or being easy to clean, for instance a suitable textile. The puoch or cover may be generally pipe-shaped and may comprise means such as hook and loop fasteners or buttons for adaptation in size.

Alternatively, the band-shaped body structure 2 may be arranged or integrated in a pillow such as to provide stimulus to the brain 26 of a subject 5 when the subject is lying down resting his or her head 25 on the pillow. The pouch or cover may thus also be that of a pillow or be adapted to be integrated in a pillow.

The drive electronics 4 are configured for driving the plurality of coils 3 by providing alternating current, AC, pulses to the plurality of coils 3.

The plurality of coils 3 are configured for, in operation and in reaction to the AC pulses received from the drive electronics 4, providing a stimulus in the form of PEMFs to the tissue, such as brain tissue, of a subject. The plurality of coils 3 is provided in and/or on the band-shaped body 2 structure. The plurality of coils 3 may for instance be embedded in the band-shaped body 2 structure. Generally, the plurality of coils 3 are distributed along a length L of the band-shaped body structure 2, such that when the band-shaped body structure 2 is mounted around the head 25 of a subject 5 (cf. Fig. 6) such that the subject 5 carries it like a head-band, particularly a head-band extending along all of the circumference of the head 25 of the subject 5, coils 3 are arranged along all of the circumference of the head 25 of the subject 5.

In the embodiment shown, the plurality of coils 3 are furthermore distributed evenly and along the entire length of the band-shaped body structure 2. In this way it is ensured that when the band-shaped body structure 2 is mounted around the head 25 of a subject 5 (cf. Fig. 6) such that the subject 5 carries it like a head-band, particularly a head-band extending along all of the circumference of the head 25 of the subject 5, coils 3 are arranged along all of the circumference of the head 25 of the subject 5.

Thereby, different coils 3 may be used to stimulate different areas or centres of the brain 26, and in particular at least one coil (cf. e.g. the coil 3" shown on Fig. 6) of the plurality of coils 3 may be used to stimulate the frontal region 27, for instance the frontal lobe of the brain 26, of the subject 5.

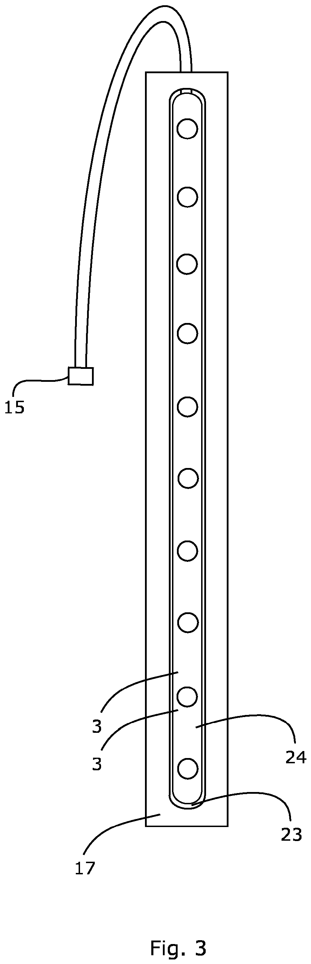

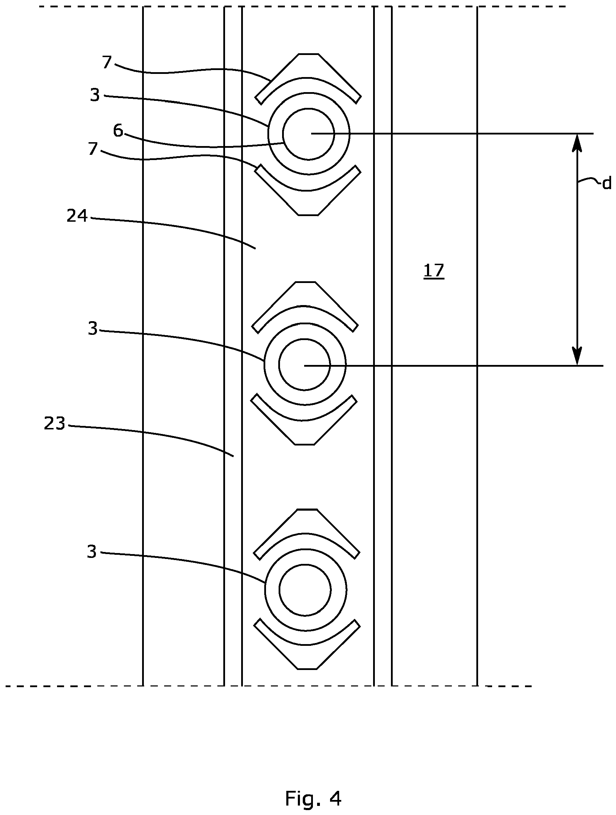

The plurality of coils 3 of the electromagnetic device 1 shown in Fig. 1 comprise twenty coils 3. In other embodiments, another number, particularly another even number, of coils 3 may be provided, such as 8, 10, 12 or 16 or 20 or more than 20 coils 3. For instance, the embodiment shown in Fig. 3 comprises ten coils 3. The coils 3 are as shown in Figs. 1 and 4 further distributed with a center to center distance d chosen to avoid unwanted interference between the coils 3. Also, the center to center distance d between neighbouring coils 3 may be chosen to be constant such that the coils 3 are evenly along the length of the band-shaped body structure 2. The coils 3 are connected electrically in such a way that, in operation, the same amount of current is provided to each coil 3, such that the functional effect of the coils 3 become evenly distributed over the area of application, such as around the head or cranium.

Stimulation of a subject's brain by means of the electromagnetic device 1 is generally carried out by sending an alternating current (AC) pulses through each coil of the plurality of coils 3. The coils 3 thereby create PEMFs which provides the stimulus of the brain 26 of a subject 5. The AC pulses may be formed in various manners. For instance, single-phase AC-pulses with a short rise time are in the art considered very effective for stimulating the brain. The AC-pulses may for instance be square pulses, such as square pulses with a period of less than one second and a rise time of less than one second. The AC-pulses may also be saw-tooth pulses. For instance, the AC-pulses may have a period in the millisecond-range, such as about 6 ms. Since the AC-pulses are of a short duration, and since the drive electronics 4 delivers an electrical effect in only about 5 % of the effective time, it becomes possible to avoid generation of heat in the band-shaped structure 2.

The intensity of the PEMFs created are decisive for the distance into the brain tissue to be treated that the PEMFs may penetrate. In particular, the higher the intensity of the PEMF's, the larger the possible penetration depth of the pulses. Thus, PEMFs with a high intensity are advantageous, especially for stimulating deeper regions of the brain 26 of a subject 5. The intensity of the PEMFs may be increased by increasing the magnitude of the AC-pulses and by increasing the number of windings of the coils 3. However, a high number of windings also provides an increased resistance of the coil and thus an increased energy loss in the coil. Therefore, the coils 3 of the electromagnetic device 1 are constructed with a low inner resistance. Alternatively, or additionally, to increase the intensity of the PEMFs the electrodes 3 may as is shown in Fig. 4 be wound on a ferrite core 6 or an iron core and/or be at least partially surrounded by a ferrite element 7 or an iron element.

The coils 3 may be provided as Brooks-coils, where the windings of the coil comprise a quadratic cross-section, and the inner diameter is two times the thickness. Other embodiments of the coils are also feasible, such as windings with circular cross-section and/or an inner diameter being smaller or larger than two times the thickness. The coils 3 may be made of copper. The coils 3 may further be provided with, such as coated or encapsulated, in a material, such as a lacquer, suitable for dampening or avoiding acoustic noise from the coils 3.

The plurality of coils 3 may be subdivided in two parts 301 and 302 forming a first half or left-side half and a second half or a right-side half of the electromagnetic device 1. The first and second part 301 and 302 may comprise the same number of coils 3, such as 5 coils or 10 coils. The part 301 extends from the middle 203 of the band-shaped body structure 2 towards the end section 201 of the band-shaped body structure 2. The part 302 extends from the middle 203 of the band-shaped body structure 2 towards the end section 202 of the band-shaped body structure 2. In that way each part 301 and 302 may be supplied by a separate amplifier and the coils 3 may work in a so-called Helmholtz-configuration in which the parts 301 and 302 are working in mutually opposite phase. Thereby, the respective two parts 310, 302 amplify one another as the total magnetic field is propagating in the same direction with a concentrated intensity and at the same time alternates in direction as controlled by the drive electronics 4. For instance, the coils 3 may be connected physically in such a way that the total electrical effect in each part 301 and 302 is in the range of 2 to 8 Ohms. If the total electrical effect in each part 301 and 302 is about 2 Ohms, the drive electronics 4 may provide short impulses with an effect of 2 x 100 Watt. If the total electrical effect in each part 301 and 302 is about 4 Ohms, the drive electronics 4 may provide short impulses with an effect of 2 x 50 Watt.

The plurality of coils 3 may alternatively be supplied by one common amplifier or amplifier channel. Such an embodiment may be used with a mono-amplifier. Using only one amplifier or amplifier channel has the advantage of using very low electrical effects, which is advantageous when aiming at treating sleep disorders.

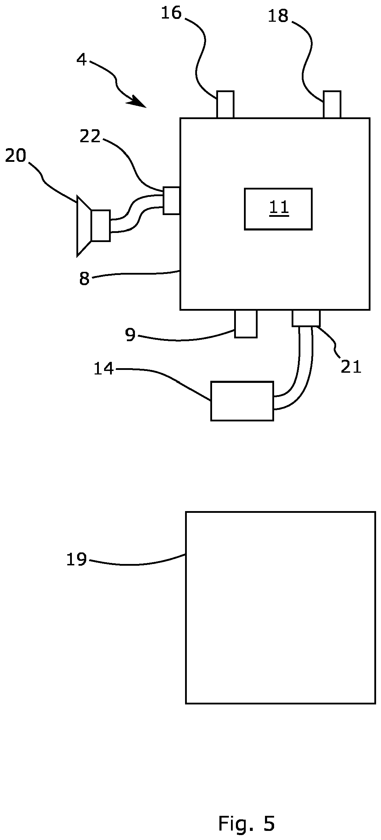

The drive electronics 4 is shown in further detail in Fig. 5. To show further details of the drive electronics 4, the top cover 19 has been removed and is shown beside the remaining parts of the drive electronics 4.

The drive electronics 4 comprises a pulse generator 12 configured to generate AC pulses, an amplifier 8 configured to amplify AC pulses, a sender/receiver unit 10 configured to send and/or receive data signals and/or AC pulses, a memory unit or memory unit input 9, a data processing device 11 and a power supply 14. An optional loudspeaker unit 20 may also be provided.

In the embodiment shown, the amplifier 8 is a class D amplifier. Other types of amplifiers may also be used. The amplifier 8 comprises inputs 18, 21 and outputs 16, 22. The output 16 are for connection to an input terminal 15 of the band-shaped body structure 2. The input 18 is for connection to an audio signal input device providing an input audio signal to the amplifier 8. The input 21 is for connection to a power supply 14. The output 22 is for connection to a loudspeaker unit 20 with which the input audio signal may be listened to. The output 22 and loudspeaker unit 20 are optional.

An exemplary cheap class D amplifier may provide an electrical effect of 50 Watt in each channel and is supplied by a 24 V power supply. The current necessary to provide the stimulating AC-pulses depend on the period and rise time of the pulses and on the amount of time between each two pulses. If for instance using pulses having a period of 6 ms and a frequency of 10 Hz when stimulating the tissue of a subject, the effect disposed in the tissue of the subject will be (10 Hz * 6 ms * 100 W) / 1000 ms or 6 Watt. Thereby, excess heat generation in the band-shaped device 2 is avoided. Such an exemplary class D amplifier could be a Texas Instruments TPA3116D2, which is capable of delivering 2 x 50 Watt at 4 Ohms and 2 x 100 Watts at 2 Ohms.

If instead using pulses having a period of 6 ms and a frequency of 3 Hz when stimulating the tissue of a subject, the effect disposed in the tissue of the subject will be (3 Hz * 6 ms * 100 W) / 1000 ms or about 2 Watt. Thereby, excess heat generation in the band-shaped device 2 is avoided and such a low effect may be provided even by means of a small, Bluetooth-based amplifier for as long as 30 minutes corresponding to the recommendations when treating sleep disorders. Such small Bluetooth-based amplifiers are often supplied by voltage sources considerably less than 24 V. In embodiments using such small Bluetooth-based amplifiers it may thus be necessary to add a voltage amplifier, for instance by means of digital electronics.

Thus, in the embodiment shown the connection 15 between the electrodes 3 and the drive electronics 4 is a cable connection. In principle the connection between the electrodes 3 and the drive electronics 4 may also be wireless.

The connection to the audio signal input device may be either wired or wireless. If wireless, the input 18 may be a receiver configured for communication with an external device, e.g. via Bluetooth or a suitable wireless network, such that the external device may supply the amplifier 8 with an audio data file comprising the needed audio input. Thereby, the drive electronics 4, and in particular the amplifier 8, may be controlled via an app on a telephone, a laptop computer, a tablet computer or the like. Alternatively, or additionally, the needed audio input may be provided on a USB stick, a SD card or like memory unit, which may be connected to the memory unit input 9 of the amplifier 8. In any event such solutions enable easy and quick updates of the electromagnetic device 1, and especially the software thereof, and also enables the provision of support to the user of the electromagnetic device 1 in a simple and straight forward manner.

In any event, the drive electronics 4, and in particular the amplifier 8, is configured to operate at voltages below 25 Volts. Furthermore, the drive electronics 4, and in particular the amplifier 8, are configured to operate at an impedance of 1 to 16 Ohms, or at an impedance of 2 to 4 Ohms or 4 to 16 Ohms. Drive electronics 4 configured to operate at an impedance of 2 to 4 Ohms are particularly advantageous since such an impedance corresponds to the impedance most often used in amplifiers for common HIFI-purposes such as loudspeakers, and since it consequently becomes straight forward to find cheap and effective amplifiers well suited for use with a device according to the invention on the market. The drive electronics 4, and in particular the amplifier 8, is further configured to provide the plurality of coils 3 with AC pulses having a frequency of less than 3 Hz, such as between 1,5 to 3 Hz. Alternatively, or additionally, the drive electronics 4, and in particular the amplifier 8, is further configured to provide the plurality of coils 3 with AC pulses having a frequency of between 45 and 60 Hz, such as between 50 to 60 Hz, for instance 55 Hz. Alternatively, or additionally, the drive electronics 4, and in particular the amplifier 8, is further configured to provide the plurality of coils 3 with AC pulses having a frequency of around 20 Hz.

Also, the drive electronics 4 may be configured to operate the plurality of coils 3 such that coils, which when the band-shaped body structure is mounted on the head 25 of a subject 5 are arranged on mutually opposite positions of the head 25 of the subject 5, such as e.g. the coils 3' on Fig. 1, are operated in mutually opposite phases.

The drive electronics 4 are in the embodiment shown arranged externally with respect to the band-shaped body structure 2 and the plurality of coils 3 and may for instance be carried in a pocket of the user.

Alternatively, the drive electronics 4 may be arranged together with the band-shaped body structure 2 and the plurality of coils 3, for instance in the same pouch or cover as the band-shaped body structure 2. The pouch or cover may for instance comprise a dedicated pocket to the drive electronics 4. The drive electronics 4 may also, together with the band-shaped body structure 2 and the electrodes 3, be incorporated in a pillow. The drive electronics may also be incorporated in the band-shaped body structure 2. This is possible due to the short periods of the stimulating pulses used mentioned above which enables avoiding heat generation in the band-shaped structure. In such embodiments, only the power supply 14 is external with respect to the band-shaped body structure 2.

The power supply 14 may be a suitable battery or power bank or a suitable connection to mains. If needed, the voltage provided by the power supply 14 may be converted to a voltage suitable for the drive electronics 4 by means of a suitable converter or transformer coupled between the power supply 14 and the drive electronics 4. In any event, the power supply 14 is configured to provide a voltage of below 24 Volts.

Turning now to Figs. 3 and 4, a mould 17 for moulding the band-shaped body structure 2 is shown. The mould 17 comprises a cavity 23, which is provided with the shape that it is desired to give the band-shaped body structure 2.

When manufacturing the band-shaped body structure 2 of the electromagnetic device 1, the desired number of coils 3 - in Fig. 3 ten coils 3 - are arranged on a substrate 24. The coils 3 may be provided with, such as coated or encapsulated, in a material, such as a lacquer, suitable for dampening or avoiding acoustic noise from the coils 3. The substrate 24 serves to keep the appropriate distance between the coils. Furthermore, the connection 15 to the drive electronics 4 is attached to the first coil. The substrate 24 with the coils 3 with or without encapsulation for avoiding acoustic noise are placed in the cavity 23 such that the connection 15 extends out of an end of the mould 17. Then, the cavity 23 of the mould 17 is filled with a suitable material for the band-shaped body structure 2, such as silicone. The material is then hardened. When the material is hardened, the band-shaped body structure 2, now ready for use, may be removed from the mould.

In the embodiment shown in Fig. 4, ferrite cores 6 are furthermore arranged on the substrate within each coil 3, and ferrite elements 7 are arranged on the substrate on the outside of each coil 3. Thereby the appropriate distance between ferrite core 6, coil 3 and ferrite elements 7 are also maintained during moulding, and the ferrite cores 6 and elements 7 are also moulded into the body-shaped structure 2.

It is noted that other moulding techniques and manufacturing techniques may also be employed to manufacture an electromagnetic device 1 according to the invention.

The person skilled in the art realizes that the present invention by no means is limited to the preferred embodiments described above. On the contrary, many modifications and variations are possible within the scope of the appended claims.

### Example 1 - treatment of depression disorders

As mentioned, treatment of depression disorders using PEMFs are medically proved to have effect. See for instance Klaus Martiny et al.: "Transcranial Low Voltage Pulsed Electromagnetic Fields in Patients with Treatment-Resistant Depression", Biol Psychiatry, 2010.68. The prior art, such as the article of Martiny et al., reveals that when treating depression disorders, stimulation with relatively high magnetic fields, such as in the order of 18 to 20 Gauss, is needed.

Calculations performed on an electromagnetic device 1 according to the invention have shown that providing the coils 3 of an electromagnetic device 1 according to the invention with a current of one Ampere will induce a magnetic field of 100 Gauss. This is five times the magnitude of the magnetic fields used in the prior art and having been shown effective in treating depression disorders.

It is thus clear that the electromagnetic device 1 according to the invention is easily and readily useful for treating depression disorders. For instance, an electromagnetic device 1 according to the invention with 20 coils divided in two parts of 10 coils each and supplied by a two channel (stereo) amplifier may be used when treating depression disorders.

### Example 2 - treatment of sleep disorders

When treating sleep disorders, previously conducted experiments have indicated that relatively low magnetic fields, such as in the order of 5 Gauss, induced by the coils 3 are sufficient to induce sleep in the subject. An electromagnetic device 1 according to the invention with 10 coils supplied by a one channel (mono) amplifier providing an electrical effect of about 3 Watts may be used when treating sleep disorders.

A number of subjects have tested an electromagnetic device according to the invention incorporated in a pillow. The subjects have all had sleep disorders, such as serious sleep interruptions on a nightly recurring basis, for several years prior to the experiments. The subjects used the device each night when going to bed. After one week of treatment, the subjects all experienced considerable improvement in their sleep pattern, including being able to sleep without interruptions.

The experiments have also shown that the effects are obtainable by mans of stimulation with a magnetic field of as low as 2 Gauss using a square pulse with a frequency of 4 Hz.

### List of reference numerals

- 1: Electromagnetic device
- 2: Band-shaped body structure
- 3: Coils
- 4: Drive electronics
- 5: Subject
- 6: Ferrite core
- 7: Ferrite element
- 8: Amplifier
- 9: Memory unit
- 10: Sender/receiver
- 11: Data processing device
- 12: Pulse generator
- 13: Pillow
- 14: Power supply
- 15: Connection to drive electronics
- 16: Connection to coils
- 17: Mould
- 18: Connection to audio input
- 19: Top cover
- 20: Speaker
- 21: Connection to power supply
- 22: Connection to speaker
- 23: Cavity of mould
- 24: Substrate
- 25: Head
- 26: Brain
- 27: Frontal region of brain

- 201: End of band-shaped body structure
- 202: End of band-shaped body structure
- 203: Middle of body structure
- 301: First part of electrodes
- 302: Second part of electrodes

- L: Length of band-shaped body structure
- W: Width of band-shaped body structure
- d: Core-to-core distance between coils

## Claims

1. An electromagnetic device adapted for stimulating tissues, particularly brain tissues, of a subject with pulsed electromagnetic fields, PEMF, with the aim of treating psychiatric disorders, such as depression disorders and/or sleep disorders, the electromagnetic device (1) comprising:
a band-shaped body structure (2), a plurality of coils (3) and drive electronics (4),
the drive electronics (4) being configured for driving the plurality of coils by providing alternating current, AC, pulses to the plurality of coils,
the plurality of coils (3) being configured for, in operation and in reaction to the AC pulses received from the drive electronics, providing a stimulus in the form of PEMFs to the tissue of a subject, wherein
the plurality of coils (3) is provided in and/or on the band-shaped body structure (2) distributed along a length (L) of the band-shaped body structure such that when the band-shaped body structure is mounted on the head of a subject in a head-band-like manner, coils are arranged along all of the circumference of the head of the subject, and such that at least one coil (3") of the plurality of coils is arranged adjacent to the frontal region of the brain of the subject such as to enable, when the coils are in operation, stimulating the frontal region of the brain of the subject, and that other coils of the plurality of coils are arranged adjacent to other regions of the brain such as to enable, when the coils are in operation, stimulating other regions of the brain of the subject, and **characterized in that**
the drive electronics (4) is configured to operate the plurality of coils (3) such that coils, which when the band-shaped body structure is mounted on the head of a subject are arranged on mutually opposite positions of the head of the subject, are operated in mutually opposite phases.

2. An electromagnetic device according to claim 1, wherein the coils of the plurality of coils (3) are distributed evenly along the length of the band-shaped body structure.

3. An electromagnetic device according to any one of the above claims, wherein the coils of the plurality of coils (3) are distributed along the entire length of the band-shaped body structure.

4. An electromagnetic device according to any one of the above claims, wherein the band-shaped body structure (2) comprises opposite end sections at longitudinally opposite ends (201; 202), and wherein no coils are arranged in the end sections.

5. An electromagnetic device according to any one of the above claims, wherein the drive electronics (4) are configured to operate at an impedance of 1 to 16 Ohms, preferably 2 to 4 Ohms or 4 to 16 Ohms, and/or at a voltage below 25 Volts.

6. An electromagnetic device according to any one of the above claims, wherein the drive electronics (4) is configured to operate the plurality of coils (3) such that coils which are spaced apart with a distance corresponding to half of a length of the band-shaped body structure are operated in mutually opposite phases.

7. An electromagnetic device according to any one of the above claims, wherein the drive electronics (4) is configured to one or more of:
- provide the plurality of coils (3) with AC-pulses in the form of any one of square pulses, particularly square impulses with a period of less than one second and a rise time of less than one second, or saw-tooth pulses,
- provide the plurality of coils (3) with AC pulses having a frequency of less than 3 Hz, particularly 1,5 to 3 Hz, and
- provide the plurality of coils (3) with AC pulses having a frequency of between 45 and 60 Hz, particularly 50 to 60 Hz, such as about 55 Hz.

8. An electromagnetic device according to any one of the above claims, wherein the band-shaped body structure (2) is made of a resilient and heat resistant material, such as silicone, and preferably wherein the plurality of coils is embedded in the resilient material.

9. An electromagnetic device according to any one of the above claims, wherein the drive electronics (4) comprises any one or more of:
a) an amplifier (8), particularly a class d amplifier, configured to amplify AC pulses,
b) a pulse generator (12) configured to generate AC pulses,
c) a sender/receiver unit (10) configured to send and/or receive data signals and/or AC pulses,
d) a memory unit (9),
e) a data processing device (11), and
f) input and/or output terminals (16, 18, 21, 22) for any one of the above items b) to d).

10. An electromagnetic device according to any one of the above claims, wherein the band-shaped body structure (2):
is configured for mounting on the head of a subject, or
is provided as a head-band.

## Patentansprüche

1. Elektromagnetische Vorrichtung, die dazu geeignet ist, Gewebe, insbesondere Hirngewebe, eines Individuums mit gepulsten elektromagnetischen Feldern, PEMF, zu stimulieren, um psychiatrische Störungen, beispielsweise depressive Störungen und/oder Schlafstörungen, zu behandeln, wobei die elektromagnetische Vorrichtung (1) umfasst:
eine bandförmige Körperstruktur (2), eine Mehrzahl von Spulen (3) und Ansteuerelektronik (4),
wobei die Ansteuerelektronik (4) zum Ansteuern der Mehrzahl von Spulen durch Bereitstellen von Wechselstromimpulsen, AC-Impulsen, für die Mehrzahl von Spulen ausgelegt ist,
die Mehrzahl von Spulen (3) in Betrieb und in Reaktion auf die von der Ansteuerelektronik empfangenen AC-Impulse zum Bereitstellen eines Stimulus in der Form von PEMFs für das Gewebe eines Individuums ausgelegt ist, wobei die Mehrzahl von Spulen (3) in und/oder auf der bandförmigen Körperstruktur (2) derart verteilt entlang einer Länge (L) der bandförmigen Körperstruktur vorgesehen ist, dass, wenn die bandförmige Körperstruktur in einer kopfbandartigen Weise auf dem Kopf eines Individuums montiert ist, Spulen entlang des gesamten Umfangs des Kopfs des Individuums angeordnet sind und derart, dass mindestens eine Spule (3") der Mehrzahl von Spulen benachbart zu der Frontregion des Gehirns des Individuums angeordnet ist, um Stimulieren der Frontregion des Individuums zu ermöglichen, wenn die Spulen in Betrieb sind, und dass andere Spulen benachbart zu anderen Regionen des Hirns angeordnet sind, um Stimulieren anderer Regionen des Gehirns des Individuums zu ermöglichen, wenn die Spulen in Betrieb sind, und **dadurch gekennzeichnet, dass**
die Ansteuerelektronik (4) dazu ausgelegt ist, die Mehrzahl von Spulen (3) derart zu betreiben, dass Spulen, die, wenn die bandförmige Körperstruktur auf dem Kopf eines Individuums montiert ist, auf zueinander gegenüberliegenden Positionen des Kopfs des Individuums angeordnet sind, in zueinander entgegengesetzten Phasen betrieben werden.

2. Elektromagnetische Vorrichtung nach Anspruch 1, wobei die Spulen der Mehrzahl von Spulen (3) gleichmäßig entlang der Länge der bandförmigen Körperstruktur verteilt sind.

3. Elektromagnetische Vorrichtung nach einem der vorhergehenden Ansprüche, wobei die Spulen der Mehrzahl von Spulen (3) entlang der gesamten Länge der bandförmigen Körperstruktur verteilt sind.

4. Elektromagnetische Vorrichtung nach einem der vorhergehenden Ansprüche, wobei die bandförmige Körperstruktur (2) gegenüberliegende Endabschnitte an in Längsrichtung gegenüberliegenden Enden (201; 202) umfasst und wobei keine Spulen in den Endabschnitten angeordnet sind.

5. Elektromagnetische Vorrichtung nach einem der vorhergehenden Ansprüche, wobei die Ansteuerelektronik (4) dazu ausgelegt ist, bei einer Impedanz von 1 bis 16 Ohm, vorzugsweise 2 bis 4 Ohm oder 4 bis 16 Ohm, und/oder einer Spannung unter 25 Volt betrieben zu werden.

6. Elektromagnetische Vorrichtung nach einem der vorhergehenden Ansprüche, wobei die Ansteuerelektronik (4) dazu ausgelegt ist, die Mehrzahl von Spulen (3) derart zu betreiben, dass Spulen, die mit einem Abstand beabstandet sind, der einer halben Länge der bandförmigen Körperstruktur entspricht, in zueinander entgegengesetzten Phasen betrieben werden.

7. Elektromagnetische Vorrichtung nach einem der vorhergehenden Ansprüche, wobei die Ansteuerelektronik (4) zu einem oder mehrerem von Folgendem ausgelegt ist:
- Versehen der Mehrzahl von Spulen (3) mit AC-Impulsen in Form eines beliebigen von Rechteckimpulsen, insbesondere Rechteckimpulsen mit einer Periode von weniger als einer Sekunde und einer Anstiegszeit von weniger als eine Sekunde, und Sägezahnimpulsen,
- Versehen der Mehrzahl von Spulen (3) mit AC-Impulsen mit einer Frequenz von weniger als 3 Hz, insbesondere 1,5 bis 3 Hz, und
- Versehen der Mehrzahl von Spulen (3) mit AC-Impulsen mit einer Frequenz von zwischen 45 und 60 Hz, insbesondere 50 bis 60 Hz, wie etwa 55 Hz.

8. Elektromagnetische Vorrichtung nach einem der vorhergehenden Ansprüche, wobei die bandförmige Körperstruktur (2) aus einem elastischen und hitzebeständigen Material, beispielsweise Silikon, hergestellt ist und wobei vorzugsweise die Mehrzahl von Spulen in das elastische Material eingebettet ist.

9. Elektromagnetische Vorrichtung nach einem der vorhergehenden Ansprüche, wobei die Ansteuerelektronik (4) eines oder mehrere von Folgenden umfasst:
a) einen Verstärker (8), insbesondere einen Verstärker der Klasse d, der zum Verstärken von AC-Impulsen ausgelegt ist,
b) einen Impulsgenerator (12), der zum Erzeugen von AC-Impulsen ausgelegt ist,
c) eine Sender-/Empfängereinheit (10) die zum Senden und/oder Empfangen von Datensignalen und/oder AC-Impulsen ausgelegt ist,
d) eine Speichereinheit (9),
e) eine Datenverarbeitungsvorrichtung (11) und
f) Eingangs- und/oder Ausgangsanschlüsse (16, 18, 21, 22) für beliebige der vorstehenden Elemente b) bis d).

10. Elektromagnetische Vorrichtung nach einem der vorhergehenden Ansprüche, wobei die bandförmige Körperstruktur (2):
dazu ausgelegt ist, auf dem Kopf eines Individuums montiert zu werden, oder
als ein Kopfband vorgesehen ist.

## Revendications

1. Dispositif électromagnétique adapté à la stimulation des tissus, notamment cérébraux, d'un sujet par des champs électromagnétiques pulsés, CEMP, dans le but de traiter des troubles psychiatriques, tels que des troubles dépressifs et/ou des troubles du sommeil, le dispositif électromagnétique (1) comprenant :
une structure corporelle en forme de bande (2), une pluralité de bobines (3) et une électronique de commande (4),
l'électronique de commande (4) étant configurée pour entraîner la pluralité de bobines en fournissant des impulsions de courant alternatif, CA, à la pluralité de bobines,
la pluralité de bobines (3) étant configurée pour, en fonctionnement et en réaction aux impulsions de courant CA reçues de l'électronique de commande, fournir un stimulus sous forme de CEMP au tissu d'un sujet,
la pluralité de bobines (3) étant prévue dans et/ou sur la structure corporelle en forme de bande (2) répartie sur une longueur (L) de la structure corporelle en forme de bande de telle sorte que lorsque la structure corporelle en forme de bande est montée sur la tête d'un sujet à la manière d'un bandeau, des bobines sont agencées sur l'ensemble de la circonférence de la tête du sujet, et de telle sorte qu'au moins une bobine (3") de la pluralité de bobines est agencée à côté de la région frontale du cerveau du sujet de manière à permettre, lorsque les bobines sont en fonctionnement, de stimuler la région frontale du cerveau du sujet, et que d'autres bobines de la pluralité de bobines sont agencées à côté d'autres régions du cerveau de manière à permettre, lorsque les bobines sont en fonctionnement, de stimuler d'autres régions du cerveau du sujet, et **caractérisé en ce que**
l'électronique de commande (4) est configurée pour faire fonctionner la pluralité de bobines (3) de telle sorte que des bobines qui, lorsque la structure corporelle en forme de bande est montée sur la tête d'un sujet, sont agencées sur des positions mutuellement opposées de la tête du sujet, fonctionnent dans des phases mutuellement opposées.

2. Dispositif électromagnétique selon la revendication 1, les bobines de la pluralité de bobines (3) étant réparties uniformément sur la longueur de la structure corporelle en forme de bande.

3. Dispositif électromagnétique selon l'une quelconque des revendications précédentes, les bobines de la pluralité de bobines (3) étant réparties sur toute la longueur de la structure corporelle en forme de bande.

4. Dispositif électromagnétique selon l'une quelconque des revendications précédentes, la structure corporelle en forme de bande (2) comprenant des sections d'extrémité opposées aux extrémités longitudinalement opposées (201 ; 202), et aucune bobine n'étant agencée dans les sections d'extrémité.

5. Dispositif électromagnétique selon l'une quelconque des revendications précédentes, l'électronique de commande (4) étant configurée pour fonctionner à une impédance de 1 à 16 Ohms, de préférence de 2 à 4 Ohms ou de 4 à 16 Ohms, et/ou à une tension inférieure à 25 Volts.

6. Dispositif électromagnétique selon l'une quelconque des revendications précédentes, l'électronique de commande (4) étant configurée pour faire fonctionner la pluralité de bobines (3) de telle sorte que des bobines qui sont espacées d'une distance correspondant à une moitié d'une longueur de la structure corporelle en forme de bande sont utilisées dans des phases mutuellement opposées.

7. Dispositif électromagnétique selon l'une quelconque des revendications précédentes, l'électronique de commande (4) étant configurée pour une ou plusieurs des actions suivantes :
- fournir à la pluralité de bobines (3) des impulsions de courant CA sous la forme d'impulsions carrées, en particulier d'impulsions carrées avec une période inférieure à une seconde et un temps de montée inférieur à une seconde, ou d'impulsions en dents de scie,
- fournir à la pluralité de bobines (3) des impulsions de courant CA ayant une fréquence inférieure à 3 Hz, en particulier de 1,5 à 3 Hz, et
- fournir à la pluralité de bobines (3) des impulsions de courant CA ayant une fréquence comprise entre 45 et 60 Hz, en particulier entre 50 et 60 Hz, par exemple environ 55 Hz.

8. Dispositif électromagnétique selon l'une quelconque des revendications précédentes, la structure corporelle en forme de bande (2) étant constituée d'un matériau résilient et résistant à la chaleur, tel que le silicone, et de préférence la pluralité de bobines étant noyée dans le matériau résilient.

9. Dispositif électromagnétique selon l'une quelconque des revendications précédentes, l'électronique de commande (4) comprenant un ou plusieurs des éléments suivants :
a) un amplificateur (8), en particulier un amplificateur de classe d, configuré pour amplifier les impulsions de courant CA,
b) un générateur d'impulsions (12) configuré pour générer des impulsions CA,
c) une unité émettrice/réceptrice (10) configurée pour envoyer et/ou recevoir des signaux de données et/ou des impulsions CA,
d) une unité de mémoire (9),
e) un dispositif de traitement des données (11), et
f) des terminaux d'entrée et/ou de sortie (16, 18, 21, 22) pour l'un des éléments b) à d) ci-dessus.

10. Dispositif électromagnétique selon l'une quelconque des revendications précédentes, la structure corporelle en forme de bande (2) :
étant configurée pour être montée sur la tête d'un sujet, ou
étant fournie sous la forme d'un bandeau.
